# EUROPÄISCHE PATENTANMELDUNG

(11) **EP 1 228 745 A1**
(43) Veröffentlichungstag der Anmeldung: **07.08.2002**
(21) Anmeldenummer: 02002151.5
(22) Anmeldetag: 29.01.2002
(51) Int. Cl.: A61K 6/10

(54) **Verwendung einer polyalkylenpolyoxidfreien und/oder polyalkylenoxidderivatfreien Silikonmasse als Abformmasse**

(30) Priorität: 05.02.2001 DE 10105357
(71) Anmelder: Heraeus Kulzer GmbH & Co.KG, 63450 Hanau (DE)
(72) Erfinder: Schaub, Matthias, Dr., 40593 Düsseldorf (DE); Nehren, Klaus-Dieter, 41539 Dormagen (DE); Wrobel, Marcus, 41468 Neuss (DE)
(74) Vertreter: Kühn, Hans-Christian

(57) **Zusammenfassung**

Verwendung einer polyalkylenoxidfreien und/oder polyalkylenoxidderivatfreien, bei Raumtemperatur durch Kondensation aushärtenden Silikonmasse aus Hydroxy-Gruppen aufweisendes Polyorganosiloxan enthaltender Basispaste und aus Vernetzer und Katalysator aus Organometallverbindung enthaltender Aktivator-Komponente als in Verpackungen zur Darreichung in Automix-Systemen darzubietende dentale Abformmasse.

## Beschreibung

Die Erfindung betrifft die Verwendung einer polyalkylenoxidfreien und/oder polyalkylenoxidderivatfreien, bei Raumtemperatur durch Kondensation aushärtenden Silikonmasse.

In DE 198 08 557 werden dentale Abformmassen auf C-Silikon-Basis offenbart, die als dentale Abformmassen in Doppelkartuschen dargereicht werden, wobei die Aktivatorkomponente zusätzlich ein Polyadditionsprodukt mit mindestens zwei Alkoxysilyl-Gruppen im Molekül enthält, damit eine ausreichende Lagerstabilität in den Kartuschen gewährleistet ist.

In DE 199 42 467 werden dentale Abformmassen auf C-Silikon-Basis zur Darreichung in Doppelkartuschen offenbart, wobei in diesem Fall eine ausreichende Lagerstabilität dadurch erreicht wird, dass die Aktivatorkomponente zusätzlich höchstens eine Alkoxysilyl-Gruppe aufweisenden Polyether und/oder ein auf Polyether basierendes höchstens eine Alkoxysilyl-Gruppe aufweisendes Polyadditionsprodukt enthält.

Nachteilig bei den oben genannten Systemen ist die Tatsache, dass die jeweils eingesetzten Aktivatoradditive sehr genau auf die jeweilige aushärtende Silikonmasse eingestellt werden müssen, damit keine unvorhergesehenen Interaktionen zwischen den Ingredenzien auftreten, die gegebenenfalls bis zu einer völligen Unbrauchbarkeit der Silikonmasse führen können.

Aus dem vorgenannten ergibt sich das Problem, die oben genannten Nachteile zumindest teilweise zu vermeiden. Insbesondere besteht das Problem darin, aushärtende Silikonmassen bereitzustellen, die trotz einer sehr hohen Lagerstabilität, insbesondere in Kartuschen, einfach herzustellen und die Interaktionen zwischen Aktivatoradditiv und dem Rest der Komponenten der auszuhärtenden Silikonmassen ausgesprochen gering sind.

Dieses Problem wird erfindungsgemäß durch eine Verwendung nach Anspruch 1 gelöst.

Überraschenderweise konnte gezeigt werden, dass es möglich ist, polyalkylenoxidfreie und/oder polyalkylenoxidderivatfreie, bei Raumtemperatur durch Kondensation aushärtende Silikonmassen aus Hydroxy-Gruppen aufweisendes Polyorganosiloxan enthaltende Basispaste und aus Vernetzer und Katalysator aus Organometallverbindung enthaltende Aktivator-Komponente als in Verpackungen zur Darreichung in Automix-Systemen, insbesondere in Kunststoff-Kartuschen und Schlauchbeuteln, die statische oder dynamische Mischer aufweisen können, darzubietende dentale Abformmassen zu verwenden.

Es ist in dieser Hinsicht völlig überraschend, dass entgegen dem aus dem Stand der Technik vorherrschenden Vorurteil, die oben aufgeführten Aktivatoradditive zu verwenden, um eine ausreichend hohe Lagerstabilität, insbesondere in Kartuschen, von bei Raumtemperatur durch Kondensation aushärtenden Silikonmassen aus Hydroxygruppen aufweisendes Polyorganosiloxan enthaltende Basispaste und aus Vernetzer und Katalysator aus Organometallverbindungen enthaltende Aktivatorkomponente als darzubietende dentale Abformmasse zu gewährleisten, es möglich ist, eben entsprechende polyalkylenoxidfreie und/oder polyalkylenoxidderivatfreie Silikonmassen als dentale Abformmassen zu verwenden.

Zunächst ist es vorteilhaft, wenn die Kunststoff-Kartuschen aus Polypropylen bestehen und/oder deren Wandstärke mindestens 0,5 mm beträgt, da sich dies in der Praxis bewährt hat.

Vernetzer und Katalysator bilden die Bestandteile der Aktivator-Komponente, wobei sich in der Praxis als vorteilhaft herausgestellt hat, dass die Aktivatorkomponente 5 bis 70 Gewichts-% Alkoxysilane, beispielsweise Tetramethoxysilan, Tetraethoxysilan, Ethylpolysilicat, Vinyltrimethoxysilan oder Mischungen davon, oder Alkoxytitanate, beispielsweise Tetraethyltitanat, Tetra-n-propyltitanat, Tetra-n-butyltitanat, oder Mischungen davon, oder Alkoxycirkonate, beispielsweise Tetra-n-propylzirconat, Tetra-n-butylzirconat, Triethanolaminzirconat, oder Mischungen davon, als Vernetzer und/oder 0,5 bis 40 Gewichts-% Organozinnverbindungen, beispielsweise Dibutylzinndilaurat, Dibutylzinndiacetat, Dioctylzinnoxid, oder Mischungen davon, als Katalysator enthält.

Insbesondere ist es von Vorteil, wenn die Aktivator-Komponente Füllstoffe und/oder Farbstoffe und/oder Hydrophobierer und/oder Geruchs- und Geschmacksstoffe enthält, um den jeweiligen Eigenschaftsanforderungen im Einzelfall gerecht zu werden. In diesem Zusammenhang haben sich insbesondere bei Raumtemperatur wachsartige oder flüssige Kohlenwasserstoffe als Hydrophobierer und/oder pyrogene Kieselsäuren als Füllstoffe bewährt.

Die nachfolgenden Beispiele dienen zur näheren Erläuterung der erfindungsgemäßen Verwendung.

### Basis-Komponente:

In einem Vakuum-Planetenmischer werden - in der angegebenen Reihenfolge - 25 Gewichts-% Hydroxy-Gruppen aufweisendes Polydimethylsiloxan mit einer Viskosität von 5000 mPas bei 23°C, 38 Gewichts-% Hydroxy-Gruppen aufweisendes Polydimethylsiloxan mit einer Viskosität von 1000 mPas bei 23°C, 31 Gewichts-% eines Gemisches aus Natrium-Aluminiumsilikat und Diatomeenerde, 5 Gewichts-% pyrogene Kieselsäure und 1 Gewichts-% organische oder anorganische Pigmente unter normalem Druck 30 Minuten lang zu einer homogenen Paste vermischt. Anschließend wird die Paste noch für 5 Minuten im Vakuum entgast. Die fertige Paste wird dann a) in Schlauchbeutel oder b) in jeweils eine der beiden Kammern von Doppelkartuschen abgefüllt.

### Aktivator-Komponente A:

In einem Vakuum-Planetenmischer werden - in der angegebenen Reihenfolge 41 - Gewichts-% Vaseline, 18% eines hochsiedenden Kohlenwasserstoffs, 16 Gewichts-% einer pyrogenen Kieselsäure und 25 Gewichts-% eines aus Tetramethoxysilan, Tetraethoxysilan und Dibutylzinndilaurat durch einstündiges Erhitzen auf 120°C und anschließendes Abkühlen auf Raumtemperatur gewonnenen Präparats unter normalem Druck 10 Minuten lang homogenen miteinander vermischt. Anschließend wird die Paste noch für 3 Minuten im Vakuum entgast. Die so erhaltene Aktivator-Komponente wird a) in Schlauchbeutel oder b) in die jeweils zweiten Kammern der Doppelkartuschen, deren erste Kammern bereits Basis-Komponente enthalten, gefüllt.

### Aktivator-Komponente B:

In einem Vakuum-Planetenmischer werden - in der angegebenen Reihenfolge - 65 Gewichts-% Vaseline, 10 Gewichts-% einer pyrogenen Kieselsäure und 25 Gewichts-% eines aus Tetramethoxysilan, Tetraethoxysilan und Dibutylzinndilaurat durch einstündiges Erhitzen auf 120°C und anschließendes Abkühlen auf Raumtemperatur gewonnenen Präparats unter normalem Druck 10 Minuten lang homogenen miteinander vermischt. Anschließend wird die Paste noch für 3 Minuten im Vakuum entgast. Die so erhaltene Aktivator-Komponente wird a) in Schlauchbeutel oder b) in die jeweils zweiten Kammern der Doppelkartuschen, deren erste Kammern bereits Basis-Komponente enthalten, gefüllt.

### Prüfung nach der Norm ISO 4823:1992:

Die Silikon-Massen I und II werden durch Vermischen von je 4 Volumen-Teilen der Basis-Paste mit einem Volumen-Teil der Aktivator Komponente A bzw. B zubereitet und nach der Norm ISO 4823:1992 geprüft.

| Silikon-Masse | I | II |
|---|---|---|
| Rückstellung nach Verformung [%] | 99,15 | 98,95 |
| Verformung unter Druck [%] | 7,95 | 7,55 |
| lineare Dimensionsänderung [%] | 0,84 | 0,78 |
| Härteprüfung Shore A nach 60 min. | 38 | 38 |
| Verarbeitungszeit [min] | 2,4 | 2,0 |
| Aushärtezeit [min] | 3,8 | 3,0 |

### Lagerstabilität:

Zur Beurteilung der Lagerstabilität wird die Basis-Komponente und die Aktivatorkomponente A unmittelbar nach Abfüllung in 4:1 PP-Kartuschen (Polypropylen) und nach 30tägiger Lagerung der befüllten Kartuschen bei 23°C, 30°C und 60°C im Volumenverhältnis 4:1 miteinander gemischt und Verarbeitungszeit und Aushärtezeit der erhaltenen Silikon-Massen bestimmt.

| | Verarbeitungszeit [min] | Aushärtezeit [min] |
|---|---|---|
| Ausgangswerte | 2,4 | 3,8 |
| n. 30 Tagen bei | | |
| 23°C | 2,4 | 4,5 |
| 30°C | 1,9 | 3,2 |
| 60°C | 2,4 | 4,5 |

## Patentansprüche

1. Verwendung einer polyalkylenoxidfreien und/oder polyalkylenoxidderivatfreien, bei Raumtemperatur durch Kondensation aushärtenden Silikonmasse aus Hydroxy-Gruppen aufweisendes Polyorganosiloxan enthaltender Basispaste und aus Vernetzer und Katalysator aus Organometallverbindung enthaltender Aktivator-Komponente als in Verpackungen zur Darreichung in Automix-Systemen darzubietende dentale Abformmasse.

2. Verwendung nach Anspruch 1, **dadurch gekennzeichnet, dass** die Silikonmasse in Kunststoff-Kartuschen oder Schlauchbeuteln dargeboten wird.

3. Verwendung nach einem der Ansprüche 1 bis 2, **dadurch gekennzeichnet, dass** die Kunststoff-Kartuschen aus Polypropylen bestehen und/oder deren Wandstärke mindestens 0,5 mm beträgt.

4. Verwendung nach einem der Ansprüche 1 bis 3, **dadurch gekennzeichnet, dass** die Aktivator-Komponente 5 bis 70 Gewichts-% Alkoxysilane oder Alkoxytitanate oder Alkoxycirkonate enthält.

5. Verwendung nach einem der Ansprüche 1 bis 4, **dadurch gekennzeichnet, dass** die Aktivator-Komponente 0,5 bis 40 Gewichts-% Organozinnverbindungen enthält.

6. Verwendung nach einem der Ansprüche 1 bis 5, **dadurch gekennzeichnet, dass** die Aktivator-Komponente Füllstoffe und/oder Farbstoffe und/oder Hydrophobierer und/oder Geruchs-und Geschmacksstoffe enthält.

7. Verwendung nach einem der Ansprüche 1 bis 6, **dadurch gekennzeichnet**, das die Aktivator-Komponente bei Raumtemperatur wachsartige oder flüssige Kohlenwasserstoffe und/oder pyrogene Kieselsäuren enthält.
